# EUROPEAN PATENT SPECIFICATION

(11) **EP 2 446 879 B1**
(45) Date of publication and mention of the grant of the patent: **12.02.2014**
(21) Application number: 10792362.5
(22) Date of filing: 25.06.2010
(51) Int. Cl.: A61K 9/14, A61K 31/41, A61P 9/12, A61K 31/4178

(54) **PHARMACEUTICAL COMPOSITION CONTAINING CARBOXYLOSARTAN AND A PRODUCTION METHOD THEREFOR**
PHARMAZEUTISCHE ZUSAMMENSETZUNG MIT CARBOXYLOSARTAN UND HERSTELLUNGSVERFAHREN DAFÜR
COMPOSITION PHARMACEUTIQUE CONTENANT DU CARBOXY-LOSARTAN ET SON PROCEDE DE PRODUCTION

(30) Priority: 25.06.2009 KR 20090057054
(43) Date of publication of application: 02.05.2012
(73) Proprietor: Jin Yang Pharm. Co., Ltd., Seoul 137-871 (KR)
(72) Inventor: CHOI, Jae-Joon, Seoul 135-533 (KR); JANG, Soung-Baek, Seoul 138-931 (KR); JEON, Hong-Ryeol, Suwon-si Gyeonggi-do 443-793 (KR); LEE, Bong-Sang, Suwon-si Gyeonggi-do 443-757 (KR); KIM, Hyun-Il, Busan 602-830 (KR)
(74) Representative: Gallagher, Kirk James
(86) International application number: PCT/KR2010/004152
(87) International publication number: WO 2010/151080

(56) References cited:
- WO-A1-99/43210
- WO-A1-2006/115834
- US-A1- 2003 104 053
- US-A1- 2006 229 350
- US-A1- 2008 096 866
- US-A1- 2010 008 956

## Description

### BACKGROUND

### Field

The present invention relates to a pharmaceutical composition containing losartan carboxylic acid (2-butyl-4-chloro-1-[(2'-(1H-tetrazol-5-yl)[1,1'-biphenyl]-4-yl)methyl]-1H-imidazole-5-carboxylic acid) having improved dissolution rate and dissolution variation, and a preparing method thereof.

### Description of Related Art

Losartan carboxylic acid (2-butyl-4-chloro-1-[(2'-(1H-tetrazol-5-yl)[1,1'-biphenyl]-4-yl)methyl]-1H-imidazole-5-carboxylic acid) represented by the following chemical formula 1 has effects of angiotensin II receptor antagonist and thus can be used for treatment of high blood pressure:

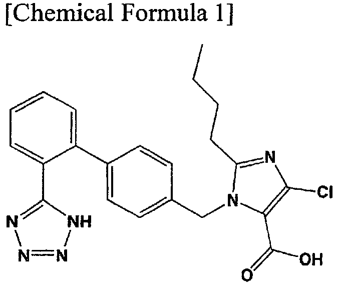

It is known that losartan carboxylic acid has effects of angiotensin II receptor antagonist that are about 10 to 14 times stronger and a half life longer than losartan, and thus can be useful as a medicine for high blood pressure (See J Hypertens, 1993 Feb 1(2):155-62 and J Chromatogr, 1992 Jan 17(2):295-301). When losartan is absorbed and then metabolized into losartan carboxylic acid, the effects of angiotensin II receptor antagonist increase. In this instance, interindividual differences in metabolic capability may result in interindividual variations in the efficacy. However, losartan carboxylic acid can fundamentally reduce the metabolic variation. Also, losartan carboxylic acid does not need metabolism and consequently has a relatively fast efficacy.

As described above, the usefulness of losartan carboxylic acid is well known, but so far studies have not been actively made on the pharmaceutical characteristics of losartan carboxylic acid.

### SUMMARY

### Technical Problem

Accordingly, it is an object of the present invention to provide a pharmaceutical composition containing losartan carboxylic acid having a variety of advantages and a preparing method thereof.

### Technical Solution

To achieve the object, the present invention provides a pharmaceutical composition containing a solid dispersion having losartan carboxylic acid represented by the following chemical formula 1 and water-soluble polymer uniformly dispersed therein:

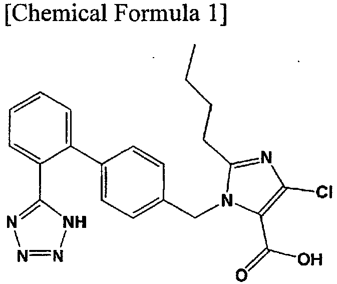

The inventors discovered through various evaluations that losartan carboxylic acid has a low dissolution speed and a low dissolution rate in a medium having a low pH, and when losartan carboxylic acid contacts water, losartan carboxylic acid agglomerates, resulting in reduced dissolution rate and a variation in dissolution. To overcome these drawbacks, the inventors suggested a solid dispersion of losartan carboxylic acid and water-soluble polymer. That is to say, the present invention is based on that a solid dispersion of losartan carboxylic acid and water-soluble polymer can solve the problems involving a low dissolution speed and a low dissolution rate of losartan carboxylic acid at a low pH. It is expected that improvement in the dissolution rate at a low pH can reduce a variation in absorption depending on the change of pH following the consumption of food and reduce the interindividual variations in absorption.

In the solid dispersion of losartan carboxylic acid and water-soluble polymer, the water-soluble polymer includes at least one selected from the group consisting of polyethylene glycol, polyoxyethylene-polyoxypropylene copolymer, and polyvinyl alcohol-polyethylene glycol copolymer. Among these water-soluble polymers, polyethylene glycol (PEG), polyethylene oxide (PEO) or polyoxyethylene (POE) can dramatically improve the dissolution rate and the initial dissolution speed in comparison with the others.

In this instance, polyethylene glycol having an average molecular weight of 6000, 4000, or 1500 may be used singularly or in combination, however the present invention is not limited in this regard. Taking the phase stability, easiness of preparation of the pharmaceutical composition and the like into account, polyethylene glycol having an average molecular weight of 6000 is the most preferred.

More preferably, the water-soluble polymer is a mixture of at least one polyethyleneglycol-based polymer selected from the group consisting of polyethylene glycol, polyoxyethylene-polyoxypropylene copolymer and polyvinyl alcohol-polyethylene glycol copolymer, and polymer other than polyethyleneglycol-based polymer. The mixture of polyethyleneglycol-based polymer and other polymer may contribute to the optimum dissolution speed and dissolution rate. Preferably, the other polymer is povidone, hydroxypropylmethylcellulose, or hydroxypropylcellulose, more preferably, povidon or hydroxypropylmethylcellulose.

The solid dispersion or the pharmaceutical composition includes silica, crospovidone, or mixtures thereof. Silica or crospovidone serves as an agent of suppressing the agglomeration of losartan carboxylic acid, thereby improving the dissolution speed and the dissolution rate, and reduces a variation in dissolution. This agglomeration suppressing agent favors the phase stability of the pharmaceutical composition containing losartan carboxylic acid.

The agglomeration suppressing agent may be included in the solid dispersion, or may be mixed with the solid dispersion after the solid dispersion is formed. To maximize the agglomeration suppressing effect, the agglomeration suppressing agent is preferably included in the solid dispersion of losartan carboxylic acid and the water-soluble solid.

In addition to the above components, the solid dispersion and/or the pharmaceutical composition according to the present invention may further include an additive, for example, an excipient, a flavoring agent, a coloring agent, a lubricant, or a filling agent, typically used to prepare a formulation for oral administration in a tablet, granule, capsule or pellet form.

The solid dispersion of the present invention may be prepared by dissolving the components together such as losartan carboxylic acid, water-soluble polymer followed by drying, or by melting the components together, followed by cooling. Alternatively, the dissolving and melting steps may be simultaneously carried out to prepare the solid dispersion of the present invention.

More specifically, the present invention also provides a method for preparing a pharmaceutical composition containing losartan carboxylic acid having improved dissolution speed and dissolution rate, the method including (S1) preparing a solution of losartan carboxylic acid and water-soluble polymer, and (S2) drying the solution to yield a solid dispersion.

A solvent for the solution may include, but is not limited to, at least one of water, ethanol, methanol, isopropyl alcohol, dichloromethane, chloroform, and acetone.

The drying may be performed by a general drying process using hot air. To maintain the mixing uniformity during the drying, agitation or shaking is preferably performed together with the drying. For mass production, a spray drying process is preferred. To perform the spray drying process, a fluidized bed granulator, a spray dryer, or a C/F instantizer may be used.

### EFFECTS OF THE INVENTION

The present invention provides a pharmaceutical composition containing losartan carboxylic acid having improved dissolution speed and dissolution rate and reduced variation in dissolution depending on the pH, and a preparing method thereof.

### DESCRIPTION OF THE DRAWINGS

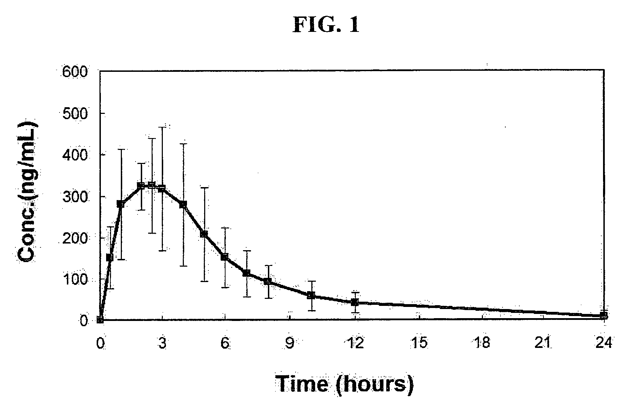

FIG. 1 is a graph illustrating the bio-absorption evaluation results of a composition containing losartan carboxylic acid having improved dissolution speed and dissolution rate.

### DETAILED DESCRIPTION

Hereinafter, the present invention will be described in detail.

### <Preparation of Comparative examples 1-4 and Example 1>

Comparative examples 1-4 and Example 1 were prepared by simply mixing losartan carboxylic acid and other components listed in Table 1 based on the contents of Table 1, and filling capsules with the mixtures.

**Table 1**

| (Unit: mg) | Comparative example 1 | Comparative Example 2 | Comparative Example 3 | Comparative Example 4 | Example 1 |
|---|---|---|---|---|---|
| Losartan carboxylic acid | 10.0 | 10.0 | 10.0 | 10.0 | 10.0 |
| Lactose | 87.0 | 77.0 | 77.0 | 77.0 | 77.0 |
| Microcrystalline cellulose | 87.0 | 77.0 | 77.0 | 77.0 | 77.0 |
| Polyethylene glycol 6000 | - | - | - | - | 10.0 |
| Povidone | - | 10.0 | - | - | - |
| HPMC | - | - | 10.0 | - | - |
| HPC | - | - | - | 10.0 | - |
| Aerosil^{®} | - | 10.0 | - | 10.0 | 10.0 |
| KOLLIDON CL-SF^{®} | - | - | 10.0 | - | - |
| Sodium carboxymethyl starch | 14.0 | 14.0 | 14.0 | 14.0 | 14.0 |
| Magnesium stearate | 2.0 | 2.0 | 2.0 | 2.0 | 2.0 |

In Table 1, HPMC is hydroxypropyl methyl cellulose, HPC is hydroxypropyl cellulose, Aerosil^{®} is the trade name of fumed silica, and KOLLIDON CL-SF^{®} is the trade name of super-fine crospovidone.

### <Evaluation of dissolution of Comparative example 1>

By using the prepared Comparative example 1, dissolution experiments was made in a buffer having a pH of 1.2 (prepared by mixing 2.0 g of sodium chloride with 7.0 mℓ of hydrochloric acid and adding purified water until it totals 1 liter), a buffer having a pH of 4.0 (prepared by mixing 0.05 mol/L acetic acid solution with 0.05 mol/L sodium acetate solution (41:9) and adjusting a pH to 4.0), a buffer having a pH of 6.8 (prepared by mixing 250 mℓ of 0.2 mol/L potassium dihydrogen phosphate test solution with 118 mℓ of 0.2 mol/L sodium hydroxide test solution and adding water until it totals 1 liter), and purified water. In this instance, a paddle method was used, an amount of a dissolution medium was 900 mℓ, and a paddle rotation rate was 50 rpm. The dissolution rate (%) over time (min) is shown in Table 2 below.

**Table 2**

| Time (min) | Dissolution rate for dissolution medium (%) | | | |
|---|---|---|---|---|
| | pH 1.2 | pH 4.0 | pH 6.8 | Purified water |
| 0 | 0.0 | 0.0 | 0.0 | 0.0 |
| 5 | 15.5 | 19.1 | 25.3 | 25.1 |
| 10 | 26.6 | 29.9 | 39.2 | 40.2 |
| 15 | 31.6 | 34.9 | 47.5 | 48.4 |
| 30 | 39.5 | 48.7 | 59.7 | 60.3 |
| 45 | 44.1 | 60.4 | 67.8 | 68.1 |
| 60 | 46.9 | 69.1 | 71.1 | 72.0 |
| 90 | 50.6 | 73.5 | 74.9 | 77.3 |
| 120 | 52.6 | 76.6 | 77.6 | 80.8 |

As shown in Table 2, it was seen that losartan carboxylic acid exhibited relatively low dissolution speed and dissolution rate in a buffer having a pH of 1.2. This means that absorption of losartan carboxylic acid in the stomach may vary with the pH change of the stomach following the consumption of food or depending on the interindividual variations in the stomach pH, and accordingly, the dissolution speed and dissolution rate of losartan carboxylic acid in a buffer having a pH of 1.2 needs to be adjusted. In subsequent experiments, a buffer having a pH of 1.2 was mainly used as a dissolution medium.

It was observed that losartan carboxylic acid slightly agglomerated during the dissolution experiment of Comparative example 1. When losartan carboxylic acid contacted purified water or a buffer, losartan carboxylic acid gelled. The gelling causes a variation in dissolution and reduces the dissolution speed and the dissolution rate, and thus needs to be controlled.

### <Evaluation of dissolution of Comparative examples 2-4 and of Example 1>

Like Comparative example 1, the dissolution of Comparative examples 2-4 and of Examples 1-4 in a buffer having a pH of 1.2 was evaluated. The results are shown in Table 3.

**Table 3**

| Time (min) | Dissolution rate (%) | | | |
|---|---|---|---|---|
| | Comparative Example 2 | Comparative Example 3 | Comparative Example 4 | Example 4 |
| 0 | 0.0 | 0.0 | 0.0 | 0.0 |
| 5 | 25.1 | 24.3 | 24.8 | **40.1** |
| 10 | 37.5 | 39.6 | 37.8 | **57.3** |
| 15 | 43.4 | 45.5 | 43.7 | **63.1** |
| 30 | 52.4 | 55.6 | 53.4 | 70.1 |
| 45 | 57.2 | 61.3 | 58.4 | 72.8 |
| 60 | 61.2 | 65.5 | 61.9 | 75.6 |
| 90 | 65.9 | 69.4 | 67.0 | 77.7 |
| 120 | 69.2 | 72.7 | 69.6 | **78.8** |

As shown in Table 3, the dissolution speed and the dissolution rate in a buffer having a pH of 1.2 was improved to some extent by simply mixing with hydrophilic polymer, and when polyethylene glycol was used as hydrophilic polymer, the improvement effects of dissolution speed and dissolution rate marked the top.

Also, agglomeration of losartan carboxylic acid in a dissolution medium was suppressed by adding fumed silica, Aerosil^{®}, or super-fine crosprovidone, KOLLIDON CL-SF^{®}, as an agglomeration suppressing agent, and it is expected that the suppression of agglomeration may improve the dissolution speed and the dissolution rate of losartan carboxylic acid.

### <Preparation of Comparative examples 5-7 and of Examples 8-10>

A solid dispersion including losartan carboxylic acid and water-soluble polymer was prepared according to the ingredients and the contents given in Table 4.

The water-soluble polymer (polyethylene glycol, povidone, HPMC, and HPC) was dissolved in an amount of purified water equivalent to 1 part by weight per the total amount of the water-soluble polymer, and ethanol was added thereto in an amount equivalent to 1 part by weight per the total amount of the purified water. Next, after losartan carboxylic acid was completely dissolved in the resulting solution, lactose, microcrystalline cellulose, and Aerosil^{®} or KOLLIDON CL-SF^{®} were added thereto, followed by uniform mixing and drying at about 40°C. Subsequently, sodium carboxymethyl starch and magnesium stearate were added thereto, and filled into a capsule.

**Table 4**

| (Unit: mg) | Comparative Example 5 | Comparative Example 6 | Comparative Example 7 | Example 8 | Example 9 | Example 10 |
|---|---|---|---|---|---|---|
| Losartan metabolome | 10.0 | 10.0 | 10.0 | 10.0 | 10.0 | 10.0 |
| Lactose | 77.0 | 77.0 | 77.0 | 77.0 | 72.0 | 72.0 |
| Microcrystalline cellulose | 77.0 | 77.0 | 77.0 | 77.0 | 72.0 | 72.0 |
| Polyethylene glycol | - | - | - | 10.0 | 10.0 | 10.0 |
| Povidone | 10.0 | - | - | - | 10.0 | - |
| HPMC | - | 10.0 | - | - | - | 10.0 |
| HPC | - | - | 10.0 | - | - | - |
| Aerosil^{®} | 10.0 | - | 10.0 | 10.0 | 10.0 | - |
| KOLLIDON CL-SF^{®} | - | 10.0 | - | - | - | 10.0 |
| Sodium carboxymethyl starch | 14.0 | 14.0 | 14.0 | 14.0 | 14.0 | 14.0 |
| Magnesium stearate | 2.0 | 2.0 | 2.0 | 2.0 | 2.0 | 2.0 |

### <Evaluation of dissolution of Comparative examples 5-7 and of Examples 8-10>

Like Example 1, the dissolution of Comparative examples 5-7 and of Examples 8-10 in a buffer having a pH of 1.2 was evaluated. The results are shown in Table 5.

**Table 5**

| Time (min) | Dissolution rate (%) | | | | | |
|---|---|---|---|---|---|---|
| | Comparative Example 5 | Comparative Example 6 | Comparative Example 7 | Example 8 | Example 9 | Example 10 |
| 0 | 0.0 | 0.0 | 0.0 | 0.0 | 0.0 | 0.0 |
| 5 | 36.1 | 31.0 | 22.3 | 44.0 | 29.4 | 26.8 |
| 10 | 56.7 | 43.0 | 35.3 | 60.2 | 53.0 | 48.4 |
| 15 | 63.7 | 54.3 | 44.4 | 67.3 | 70.1 | 62.8 |
| 30 | 74.3 | 67.9 | 61.0 | 77.1 | 87.7 | 81.3 |
| 45 | 83.0 | 74.9 | 74.7 | 81.6 | 93.8 | 88.1 |
| 60 | 87.7 | 83.8 | 78.2 | 84.7 | 95.7 | 91.5 |
| 90 | 90.7 | 90.5 | 83.1 | 88.9 | 99.0 | 97.3 |
| 120 | 92.2 | 91.8 | 87.5 | 92.6 | 101.4 | 99.8 |

As shown in Table 5, it was found that the preparation of a solid dispersion improved the dissolution rate on the whole. In particular, Example 9 showed that about 90% of losartan carboxylic acid was discharged in about 30 minutes after the start of dissolution, and thus exhibited the improved dissolution speed in comparison with the other examples. From the above dissolution results, it was estimated that polyethylene glycol improved the initial dissolution speed and the total dissolution rate, and the water-soluble polymer used with polyethylene glycol improved the dissolution rate at the end of dissolution, however the present invention is not limited in this regard.

### <Evaluation of dissolution for dissolution medium in Example 9>

Like Comparative example 1, the dissolution of Example 9 in different dissolution media was evaluated. The results are shown in Table 6.

**Table 6**

| Time (min) | Dissolution rate for dissolution medium (%) | | | |
|---|---|---|---|---|
| | pH 1.2 | pH 4.0 | pH 6.8 | Purified water |
| 0 | 0.0 | 0.0 | 0.0 | 0.0 |
| 5 | 43.3 | 69.8 | 71.8 | 68.2 |
| 10 | 59.9 | 91.2 | 98.0 | 96.9 |
| 15 | 75.1 | 97.4 | 100.1 | 98.4 |
| 30 | 88.7 | 101.0 | 101.3 | 100.1 |
| 45 | 91.7 | 99.8 | 101.8 | 100.8 |
| 60 | 94.7 | 100.2 | 102.6 | 100.3 |
| 90 | 95.9 | 100.6 | 102.7 | 100.6 |
| 120 | 98.3 | 100.8 | 102.5 | 101.2 |

As shown in Table 6, it was found that Example 9 completed dissolution in all the dissolution media in a short time, and had the improved dissolution rate in comparison with Comparative example 1. It is expected that this improvement can speed up the absorption of losartan carboxylic acid after administration, leading to a more efficient drug delivery. Also, it is expected to reduce the variations in dissolution speed and dissolution rate depending on the pH, thereby considerably reducing the variations in absorption within an individual and between individuals that may occur under the influence of food and the like.

### <Evaluation of bio-absorption of Example 9>

In the same way of Example 9, an experiment for absorption evaluation was made using a tablet containing 20 mg of losartan carboxylic acid for each tablet. The number of subjects was six, and a tablet containing losartan carboxylic acid was taken on an empty stomach together with 240 mL of water. The concentration in blood of losartan carboxylic acid was measured using LC/MS/MS. The results are shown in Table 1.

As shown in Table 1, the composition containing losartan carboxylic acid according to the present invention showed good absorption. The composition of Example 9 had a mean Cmax of 374.2 ng/ml, a mean Tmax of 1.8 hr, and an AUC (last) of about 2209.4.

## Claims

1. A pharmaceutical composition containing a solid dispersion of losartan carboxylic acid represented by the following chemical formula 1 and water-soluble polymer: 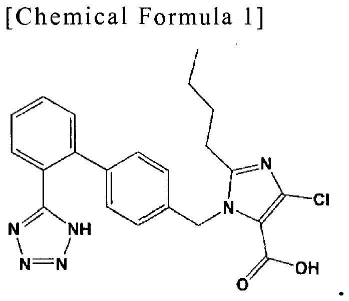 wherein the solid dispersion is made by preparing a solution of losartan carboxylic acid and water-soluble polymer, and drying the solution;
the water-soluble polymer includes at least one polyethylene glycol-based water-soluble polymer selected from the group consisting of polyethylene glycol, polyoxyethylene-polyoxypropylene copolymer, and polyvinyl alcohol-polyethylene glycol copolymer; and
the pharmaceutical composition or the solid dispersion comprises silica, crospovidone, or mixtures thereof.

2. The pharmaceutical composition according to claim 1,
wherein the water-soluble polymer is a mixture of at least one polyethylene glycol-based water-soluble polymer selected from the group consisting of polyethylene glycol, polyoxyethylene-polyoxypropylene copolymer, and polyvinyl alcohol-polyethylene glycol copolymer, and water-soluble polymer other than the polyethylene glycol-based water-soluble polymer.

## Patentansprüche

1. Pharmazeutische Zusammensetzung, enthaltend eine feste Dispersion von Losartancarbonsäure, repräsentiert durch die folgende chemische Formel 1, und wasserlöslichem Polymer: 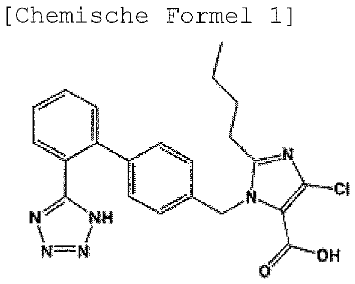 wobei die feste Dispersion durch Zubereiten einer Lösung von Losartancarbonsäure und wasserlöslichem Polymer und Trocknen der Lösung hergestellt wird;
das wasserlösliche Polymer wenigstens ein wasserlösliches Polymer auf Polyethylenglykolbasis beinhaltet, das aus der aus Polyethylenglykol, Polyoxyethylen-Polyoxypropylen-Copolymer und Polyvinylalkohol-Polyethylenglykol-Copolymer bestehenden Gruppe ausgewählt ist; und
die pharmazeutische Zusammensetzung bzw. die feste Dispersion Siliciumdioxid, Crospovidon oder Gemische davon umfasst.

2. Pharmazeutische Zusammensetzung nach Anspruch 1, wobei es sich bei dem wasserlöslichen Polymer um ein Gemisch wenigstens eines wasserlöslichen Polymers auf Polyethylenglykolbasis, das aus der aus Polyethylenglykol, Polyoxyethylen-Polyoxypropylen-Copolymer und Polyvinylalkohol-Polyethylenglykol-Copolymer bestehenden Gruppe ausgewählt ist, und von dem wasserlöslichen Polymer auf Polyethylenglykolbasis verschiedenen wasserlöslichen Polymers handelt.

## Revendications

1. Composition pharmaceutique contenant une dispersion solide d'acide carboxylique losartan représenté par la formule chimique 1 suivante et de polymère hydrosoluble : 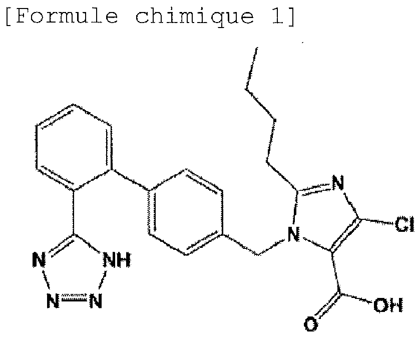 dans laquelle la dispersion solide est fabriquée en préparant une solution d'acide carboxylique losartan et de polymère hydrosoluble, et en séchant la solution ;
le polymère hydrosoluble comprend au moins un polymère hydrosoluble à base de polyéthylèneglycol choisi dans le groupe constitué de polyéthylèneglycol, copolymère de polyoxyéthylène-polyoxypropylène, et copolymère de poly(alcool vinylique)-polyéthylèneglycol ; et
la composition pharmaceutique ou la dispersion solide comprend de la silice, de la crospovidone, ou des mélanges de celles-ci.

2. Composition pharmaceutique selon la revendication 1,
dans laquelle le polymère hydrosoluble est un mélange d'au moins un polymère hydrosoluble à base de polyéthylèneglycol choisi dans le groupe constitué de polyéthylèneglycol, copolymère de polyoxyéthylène-polyoxypropylène, et copolymère de poly(alcool vinylique)-polyéthylèneglycol, et un polymère hydrosoluble autre que le polymère hydrosoluble à base de polyéthylèneglycol.
